# EUROPÄISCHE PATENTSCHRIFT

(11) **EP 2 170 434 B1**
(45) Veröffentlichungstag und Bekanntmachung des Hinweises auf die Patenterteilung: **23.05.2012**
(21) Anmeldenummer: 08757281.4
(22) Anmeldetag: 26.06.2008
(51) Int. Cl.: A61M 5/20

(54) **INJEKTIONSVORRICHTUNG MIT EINER FEDER FÜR EINE NADELSCHUTZHÜLSE**
INJECTION DEVICE WITH A SPRING FOR A NEEDLE GUARD SLEEVE
DISPOSITIF D'INJECTION COMPRENANT UN RESSORT POUR UNE GAINE DE PROTECTION D'AIGUILLE

(30) Priorität: 29.06.2007 DE 102007030327
(43) Veröffentlichungstag der Anmeldung: 07.04.2010
(73) Patentinhaber: Tecpharma Licensing AG, 3401 Burgdorf (CH)
(72) Erfinder: HOMMANN, Edgar, CH-3257 Grossaffoltern (CH); SCHERER, Benjamin, CH-8610 Uster (CH); THOMPSON, Ian, CH-3400 Burgdorf (CH)
(86) Internationale Anmeldenummer: PCT/CH2008/000288
(87) Internationale Veröffentlichungsnummer: WO 2009/003300

(56) Entgegenhaltungen:
- WO-A-94/21316
- WO-A-2005/023341
- WO-A-2005/044344
- WO-A-2005/044347
- DE-A1-102004 060 146

## Beschreibung

Die Erfindung betrifft eine Injektionsvorrichtung, wie zum Beispiel einen Autoinjektor zum injizieren eines flüssigen Produkts, wie zum Beispiel eines Medikaments zur Diabetes- oder Insulintherapie. Bei der Injektionsvorrichtung wird nach erfolgter Produktverabreichung bevorzugt eine Nadelschutzhülse über eine Injektionsnadel zum Schutz vor Verletzungen geschoben.

Aus dem Stand der Technik ist bekannt, die Nadelschutzhülse mittels einer Feder aus ihrer proximalen Position, in der die Nadel freiliegt, in distale Richtung über die Nadel zu schieben, so dass die Nadel vor äußeren Zugriffen geschützt wird. Die den Nadelschutz mit einer Federkraft beaufschlagende Feder ist in der proximalen Position der Nadelschutzhülse um einen Betrag vorgespannt. Beim Vorschieben der Nadelschutzhülse über die Injektionsnadel nimmt die Federkraft nach den Regeln des Hookeschen Gesetzes ab. Die Vorspannung der Feder muss daher so gewählt werden, dass die Nadelschutzhülse die Endposition in der sie die Injektionsnadel verdeckt, sicher einnehmen kann.

Ferner ist es bekannt, die Nadelschutzhülse zuvor aus einer distalen Position in die proximale Position zu verschieben, um die Feder vorzuspannen. Bei dieser Bewegung werden ggf. mechanische Schaltvorgänge von mit der Nadelschutzhülse gekoppelten Teilen ausgeführt. Um diese Schaltvorgänge zu bewirken, werden zusätzlich zu der zum Vorspannen der Feder benötigten Kraft, weitere in proximale Richtung auf die Nadelschutzhülse wirkende Kräfte zum Verschieben der Nadelschutzhülse in proximale Richtung benötigt. Es ist jedoch wünschenswert, die zum Verschieben der Nadelschutzhülse in proximale Richtung benötigte Gesamtkraft niedrig zu halten, um dem Anwender die Handhabung der Vorrichtung zu erleichtern. Andererseits werden - wie weiter oben beschrieben wird - erhöhte Federkräfte zum Verschieben der Nadelschutzhülse aus der proximalen Position in distale Richtung gewünscht.

Aus der, WO2005/023341A1, DE102004060146A1 und WO2005/044344A1 sind Injektionsvorrichtung, insbesondere Autoinjektoren bekannt, die eine Nadelschutzhülse aufweisen, die sich an einer ungespannten Nadelschutzfeder abstützt. Während des Einstechvorgangs der Nadel wird die Nadelschutzfeder gespannt. Nach Abnahme der Injektionsvorrichtung von der Einstechstelle kann die nun gespannte Feder die Nadelschutzhülse distal über die Nadelspitze schieben.

Des Weiteren zeigt WO2005/044347A1 eine Injektionsvorrichtung, die eine Nadelschutzhülse aufweist, welche auf einem Sprilzenhalter innerhalb der Injektionsvorrichtung gelagert ist um eine Verschiebung in distale Richtung zu verhinder und einen Auslöseknopf, wobei über eine axiale Verschiebung der Nadelschutzhülse in proximale Richtung ein Koppelelement verschoben wird, so dass der Auslöseknopf seitlich zur Mittelachse hin gedrückt und die Injektion ausgelöst werden kann. Nach dem Eindrücken des Auslöseknopfes wird die Spritze mit der Nadel in eine vordere Position geschoben, wobei die zwischen dem Spritzenhalter und dem Nadelschutz angeordnete Nadelschutzfeder gespannt wird.

Es ist daher eine Aufgabe der Erfindung, eine Injektionsvorrichtung und ein Verfahren bereitzustellen, bei denen die Federkraft der Nadelschutzhülse dem Verabreichungsvorgang entsprechend adaptierbar ist.

Es ist eine weitere Aufgabe der Erfindung das Sperren des Auslöseelements und das Halten der Nadelschutzhülse sicher zu gewährleisten.

Die Aufgabe wird gelöst durch die Merkmale der unabhängigen Ansprüche. Vorteilhafte Weiterentwicklungen ergeben sich aus den abhängigen Ansprüchen.

Die Erfmdung betrifft eine Injektionsvorrichtung zum Verabreichen eines flüssigen Produkts. Bei dem flüssigen Produkt kann es sich zum Beispiel um Insulin, ein Hormon oder ein anderes Medikament handeln. Die Injektionsvorrichtung kann als einfache Injektionsvorrichtung ausgestaltet sein, d.h., dass das Einstechen einer an der Injektionsvorrichtung gebildeten Injektionsnadel von Hand vorgenommen wird. Alternativ kann die Injektionsvorrichtung als Autoinjektor ausgestaltet sein, bei der das Einstechen der Injektionsnadel durch einen Einstechmechanismus erfolgt, wobei nach dem Einstechen der Injektionsnadel über einen Ausschüttmechanismus eine Produktausschüttung erfolgt.

Die Injektionsvorrichtung weist ein Gehäuse auf. Das Gehäuse kann einteilig sein. Bevorzugt ist, dass das Gehäuse mehrteilig ist und beispielsweise ein proximales Gehäuseteil und ein damit axial fest verrastetes distales Gehäuseteil umfasst. Ein mehrteiliges Gehäuse bildet Vorteile bei der Montage der Injektionsvorrichtung.

Die Injektionsvorrichtung umfasst ferner ein relativ zum Gehäuse verschiebbares Betätigungselement, das allgemein bevorzugt eine Nadelschutzhülse ist. Das Betätigungselement kann vorzugsweise entlang der Längsachse des beispielsweise zylinderförmigen Gehäuses bewegbar sein. Insbesondere kann das Betätigungselement zwischen distalen und proximalen Positionen verschiebbar sein. Bevorzugt ist das Betätigungselement zumindest aus einer Betätigungsposition, insbesondere einer proximalen Position, in der das Betätigungselement z.B. betätigt ist, zum Beispiel in distale Richtung, in eine Endposition verschiebbar. Vorzugsweise kann das Betätigungselement zusätzlich aus einer Anfangsposition proximal in die Betätigungsposition verschoben, werden. Anfangsposition und Endposition können identisch sein, müssen es aber nicht. Vorzugsweise nimmt das Betätigungselement in der Endposition eine distalere Stellung ein als in der Anfangsposition. Besonders bevorzugt steht das Betätigungselement in seiner Endposition distal über das distale Ende des Gehäuses, wobei zum Beispiel das Betätigungselement in seiner Endposition distal um einen größeren Betrag über das distale Ende des Gehäuses steht als in seiner Anfangsposition. Wenn das Betätigungselement distal über das distale Ende des Gehäuses steht bildet es gleichzeitig das distale Ende der Injektionsvorrichtung. Gegebenenfalls steht das Betätigungselement auch in seiner Anfangsposition distal über das distale Ende des Gehäuses. Aus dieser Position kann es beispielsweise in die Betätigungsposition bewegbar sein.

Es ist bevorzugt, dass das Betätigungselement in der Endposition über das distale Ende der an der Injektionsvorrichtung gebildeten Injektionsnadel steht. Hierdurch wird ein Zugriff auf die Injektionsnadel verhindert und die Verletzungsgefahr verringert. Beispielsweise kann das Betätigungselement auch in der Anfangsposition über das distale Ende der Injektionsnadel stehen. Alternativ kann in der Anfangsposition des Betätigungselements die Injektionsnadel mit ihrem distalen Ende über das distale Ende des Betätigungselements stehen. In der Betätigungsposition des Betätigungselements kann die Injektionsnadel über das distale Ende der Injektionsvorrichtung hervorstehen. In dieser Position wird das distale Ende durch das distale Ende des Gehäuses und/oder durch das distale Ende des Betätigungselements gebildet. Vorzugsweise kann die Injektionsnadel, wenn sich das Betätigungselement in der Betätigungsposition befindet, so weit über das distale Ende der Injektionsvorrichtung stehen oder vorgeschoben werden, dass die über das distale Ende der Injektionsvorrichtung hervorragende Nadellänge der Einstechtiefe entspricht. Das Betätigungselement ist dafür vorgesehen, an eine am Patienten vorgesehene Injektionsstelle angesetzt zu werden. Beim Andrücken der angesetzten Injektionsvorrichtung an die Injektionsstelle ist es bevorzugt, dass das Betätigungselement zum Beispiel aus der Anfangsposition in die Betätigungsposition, d.h., hier in die proximale Position, verschoben wird. Hierbei kann zum Beispiel von einer Betätigung des Betätigungselements gesprochen werden. Das Betätigungselement kann dafür vorgesehen sein, dass es der Mechanik zum Einstechen der Injektionsnadel oder zum Ausschütten des Produkts anzeigt, d.h. zum Beispiel eine mechanische Schaltung bewirkt, dass die Injektionsvorrichtung ordnungsgemäß an die Injektionsstelle angesetzt und die Nadel eingestochen wurde. Besonders bevorzugt ist das Betätigungselement eine Nadelschutzhülse, welche den Zugriff auf die Nadel und damit die Verletzungsgefahr verringern kann.

Die Injektionsvorrichtung umfasst ferner eine Feder, wie zum Beispiel eine Rückstellfeder, wobei die Feder mit dem Betätigungselement so gekoppelt ist, dass mit bzw. von ihr das Betätigungselement aus der Bestätigungsposition in die Endposition verschiebbar ist. Die Feder kann eine Zugfeder oder vorzugsweise eine Druckfeder sein. Für die Feder in Frage kommenden Materialien sind Kunststoffe oder Metalle, wie zum Beispiel Federstahl. Die Feder ist vorzugsweise wendelförmig. Anstatt einer Feder können auch andere Federelemente Verwendung finden. Vorzugsweise folgen die Federelemente dem Hookeschen Gesetz, was jedoch für die Funktion der Injektionsvorrichtung nicht unbedingt erforderlich ist. Die Feder kann mit dem Betätigungselement mittelbar oder unmittelbar gekoppelt sein. Bei einer unmittelbaren Kopplung stützt sich die Feder an dem Betätigungselement ab. Bevorzugt stützt sich das distale Ende der Feder an dem Betätigungselement ab. Das andere Ende kann sich bevorzugt an einem Vortriebsglied abstützen. Bei einer mittelbar mit dem Betätigungselement gekoppelten Feder kann das Betätigungselement über ein mit dem Betätigungselement verbundenen Element gekoppelt sein, an dem sich die Feder abstützt. Ein solches Element kann zum Beispiel ein Übertragungselement sein. Das Betätigungselement und das Übertragungselement können axial fest miteinander verbunden sein. Es ist bevorzugt, dass zumindest das Betätigungselement und das Übertragungselement jeweils auf das andere eine Druckkraft übertragen können.

Das Vortriebsglied ist relativ zum Gehäuse verschiebbar. Vorzugsweise ist das Vortriebsglied in eine Vortriebsrichtung, wie zum Beispiel eine Einstechrichtung oder Ausschüttrichtung oder distale Richtung verschiebbar. Die Erfindung zeichnet sich dadurch aus, dass die Feder so mit dem Vortriebsglied gekoppelt ist, dass sie bei einer Bewegung des Vortriebsglieds in Vortriebsrichtung spannbar ist. Mit der Bewegung des Vortriebsglieds in Vortriebsrichtung wird durch das Spannen der Feder die Federkraft und damit die Kraft, welche auf das Betätigungselement wirkt, erhöht, so dass bei anfänglich geringer Kraft auf das Betätigungselement nun eine erhöhte Kraft auf das Betätigungselement wirkt. Die anfänglich geringe Kraft ist zum Beispiel für eine Betätigung, d.h. eine Bewegung des Betätigungselements in seine Betätigungsposition bzw. proximale Richtung, erwünscht. Durch die erfindungsgemäße Ausgestaltung wird der Federkraftanteil der zum Betätigen des Betätigungselements erforderlichen Gesamtkraft, die z.B. noch Schalt- oder Reibungskräfte enthalten kann, gering gehalten. Die nach dem Verschieben des Vortriebsglieds erhöhte Kraft ist erwünscht, um das Betätigungselement aus seiner Betätigungsposition vollständig und sicher in seine Endposition zu bewegen. Besonders bevorzugt ist das Betätigungselement in seiner Endposition relativ zum Gehäuse axial fest verrastet. Zum Herstellen dieser Rastverbindung, die zum Beispiel zwischen dem Gehäuse und dem Betätigungselement stattfinden kann, ist die erhöhte Federkraft ebenfalls von Vorteil, da ein sicheres Einrasten ermöglicht wird. Durch das Verrasten wird die Verletzungsgefahr noch besser verringert, da ein versehentliches Zurückschieben des Betätigungselements, was zu einem Hervortreten der Injektionsnadel führen würde, verhindert wird.

Die Vorrichtung umfasst ferner eine weitere Feder, insbesondere eine Vortriebsfeder zur Bewegung des Vortriebsglieds in Vortriebsrichtung. Die Vortriebsfeder kann die Feder des Betätigungselements spannen, die nun zur besseren Unterscheidbarkeit als Rückstellfeder bezeichnet wird. Besonders bevorzugt wird, dass die Vortriebsfeder so stark vorgespannt ist, dass sie eine größere Federkraft aufweist als die Rückstellfeder. Insbesondere sollte die Vorspannung der Vortriebsfeder so gewählt sein, dass die Kraft der Vortriebsfeder in allen Schaltpositionen der Injektionsvorrichtung größer ist als die Kraft der Rückstellfeder.

Die Injektionsvorrichtung umfasst ferner ein Produktbehältnis, das an dem Gehäuse gebildet wird. Das Produktbehältnis kann durch das Gehäuse selbst oder durch ein in das Gehäuse einlegbares oder eingelegtes separates Produktbehältnis, wie zum Beispiel eine Ampulle gebildet sein. Das Produktbehältnis ist an einem Ende mit der Injektionsnadel verbunden oder verbindbar. Vorzugsweise ist die Injektionsnadel unmittelbar am Produktbehältnis befestigt oder befestigbar. Am anderen Ende, insbesondere am proximalen Ende weist das Produktbehältnis eine Öffnung auf, die von einem im Produktbehältnis angeordneten Kolben verschlossen wird. Der Kolben ist für eine Produktausschüttung relativ zum Produktbehältnis verschiebbar, so dass das zwischen dem Kolben und dem gegenüberliegenden Ende des Produktbehältnisses befindliche Produkt aufgrund der Kolbenbewegung ausgeschüttet wird. Das Vortriebsglied kann so mit dem im Produktbehältnis angeordneten Kolben gekoppelt sein, dass dieser von dem Vortriebsglied relativ zum Gehäuse der Injektionsvorrichtung verschiebbar ist. Das Vortriebsglied kann somit mittelbar, wie zum Beispiel über das Produktbehältnis, oder unmittelbar auf den Kolben wirken.

Bevorzugt ist die Rückstellfeder bei der Bewegung des Betätigungselements aus der Anfangsposition in die Betätigungsposition oder proximale Position um einen ersten Weg spannbar und bei der Bewegung des Vortriebsglieds aus seiner Ausgangsposition, wie zum Beispiel seiner proximalen Position, in distale Richtung, insbesondere zusätzlich zum ersten Weg um einen zweiten Weg spannbar. Der erste Weg ist vorzugsweise proportional zu einem ersten Betrag der Änderung der Federkraft und der zweite Weg ist vorzugsweise proportional zu einem zweiten Betrag der Änderung der Federkraft. Somit beträgt die auf das Betätigungselement wirkende Kraft zumindest die Summe aus dem ersten Betrag und dem zweiten Betrag, wobei noch die ursprüngliche Vorspannung der Feder hinzukommt.

In einer bevorzugten Ausführungsform ist das Vortriebsglied eine Kolbenstange, die nicht zwingend massiv ausgeführt sein braucht, sondern in bevorzugten Ausführungsformen hülsenförmig, d.h. zumindest teilweise hohlzylindrisch ist. Mit dem Vortriebsglied ist der Kolben relativ zum Produktbehältnis verschiebbar. Vorzugsweise wird bei dieser Ausführungsform die Nadel per Hand in den Patienten eingestochen. Zum Beispiel kann sich die Vortriebsfeder, welche in diesem Fall eine Ausschüttfeder ist, an dem Gehäuse oder einem gehäusefesten Element und an der Kolbenstange abstützen. Ferner kann sich die Rückstellfeder an der Kolbenstange oder einem mit ihr axial fest verbundenem Element und dem Betätigungselement abstützen. Hierdurch wird bei einer Produktausschüttung die Ausschüttfeder zum Vortrieb des Kolbens entspannt, wobei gleichzeitig die Rückstellfeder von der Ausschüttfeder gespannt wird. Zum Beispiel kann ein Auslöseelement vorgesehen sein, welches eine Sperrverbindung, welche eine Bewegung der Kolbenstange in Ausschüttrichtung durch die vorgespannte Ausschüttfeder verhindert, löst, so dass eine Ausschüttbewegung stattfinden kann. Zum Beispiel kann es vorteilhaft sein, dass das Betätigungselement zusätzlich zum Auslöseelement betätigt sein muss, damit die Sperrverbindung lösbar ist. Die Elemente sind dementsprechend miteinander gekoppelt.

In einer anderen bevorzugten Ausführungsform ist die Injektionsvorrichtung ein Autoinjektor. Das Vortriebsglied kann ein Vorschubelement sein, mit dem das gesamte Produktbehältnis relativ zum Gehäuse verschiebbar ist. Durch die Verschiebung des Produktbehältnisses in eine Einstechrichtung, zum Beispiel die distale Richtung, kann die Injektionsnadel aus dem distalen Ende der Injektionsvorrichtung hervortreten, um zum Beispiel in den Patienten eingestochen zu werden. Insbesondere ist das Produktbehältnis aus einer Anfangsposition distal in eine Endposition verschiebbar. In der Endposition steht die zum Beispiel am Produktbehältnis angeordnete Injektionsnadel distal über das distale Ende der Injektionsvorrichtung hervor. Bevorzugt steht in der Anfangsposition des Produktbehältnisses die Injektionsnadel nicht über das distale Ende der Injektionsvorrichtung hervor, d.h. insbesondere, dass das distale Ende der Nadel sich proximal des distalen Endes der Injektionsvorrichtung befindet.

Das Vorschubelement kann beispielsweise durch die Vortriebsfeder, die als Einstechfeder dient, in Einstechrichtung verschoben werden. Bevorzugt kann das Vorschubelement das Produktbehältnis zumindest in distale Richtung mit einer Vorschubkraft beaufschlagen. Das Produktbehältnis kann zum Beispiel in einem Produktbehältnishalter gehalten sein, der zusammen mit dem Produktbehältnis in Einstechrichtung bewegbar ist.

Die Vortriebsfeder stützt sich in dieser Ausführungsform bevorzugt an dem Gehäuse oder einem mit dem Gehäuse axial fest verbundenen Element und an dem Vorschubelement oder einem mit dem Vorschubelement axial fest verbundenen Element ab. Bevorzugt ist das Vorschubelement in einer Position, in der sich das Produktbehältnis in seiner Ausgangsposition befindet, relativ zum Gehäuse axial fest verrastet. Beim Lösen dieser Rastverbindung wird das Vorschubelement für eine Vorschubbewegung freigegeben. Solange die Rastverbindung besteht, ist die Vortriebsfeder in einem gespannten Zustand, wobei sich bei gelöster Rastverbindung die Rückstellfeder entspannen kann und somit das Vorschubelement in Vorschubrichtung bewegt und gleichzeitig die Rückstellfeder um einen Betrag, insbesondere den zweiten Betrag spannt. Vorzugsweise ist die Rastverbindung mit der das Vorschubelement gehalten wird mit der Betätigung eines Auslöseelements, wie zum Beispiel eines Auslöseknopfs lösbar, insbesondere in Kombination mit der Betätigung der Betätigungshülse, wodurch die Rückstellfeder zum Beispiel um einen ersten Betrag vorgespannt werden kann.

Die Injektionsvorrichtung kann beispielsweise eine Kolbenstange umfassen, die relativ zum Vorschubelement verschiebbar ist. Ferner kann der Kolben von der Kolbenstange relativ zum Produktbehältnis verschiebbar sein. Besonders bevorzugt kann die Injektionsvorrichtung ein Freigabeglied umfassen, welches die Relativbewegung zwischen Kolbenstange und Vorschubelement sperren kann. Vorzugsweise sperrt das Freigabeglied die Relativbewegung zwischen Kolbenstange und Vorschubelement während der Einstechbewegung. Zum Beispiel kann ein Schaltring vorgesehen sein, der am Ende der Einstechbewegung das Freigabeglied löst oder aus dem Eingriff mit der Kolbenstange bewegt, oder zumindest ein Lösen des Freigabeglieds ermöglicht, so dass die Kolbenstange eine Ausschüttbewegung ausführen kann. Bevorzugt ist zum Vorschub der Kolbenstange, d.h. für die Ausschüttbewegung, nämlich dann, wenn sich die Kolbenstange relativ zum Vorschubelement bewegen soll, eine Ausschüttfeder vorgesehen, die auf die Kolbenstange wirkt. Zum Beispiel kann die Kolbenstange hülsenförmig sein, wobei die Ausschüttfeder sich in der hülsenförmigen Kolbenstange in einem vorgespannten Zustand befinden kann. Insbesondere kann sich die Vorschubfeder an der Kolbenstange und an einem gehäusefesten Element oder noch bevorzugter an dem Vorschubelement oder an einem mit dem Vorschubelement axial fest verbundenen Element abstützen.

Die Erfindung betrifft ferner ein Verfahren zum Betrieb einer Injektionsvorrichtung, wobei ein Vorschubglied für eine Einstechbewegung und eine Produktausschüttung relativ zu einem Gehäuse in distale Richtung bewegt wird und dabei eine auf ein Betätigungselement wirkende Feder, insbesondere Rückstellfeder spannt, wobei das Betätigungselement von der gespannten Feder aus der proximalen Position distal in eine Endposition verschoben wird und wobei ein Auslöseelement die Verabreichung des Produkts auslöst, wobei ein Übertragungselement mit einem Sperrglied in eine Sperrnut eingreift und eine Bewegung des Betätigungselements und des Auslöseelements in distale Richtung verhindert. Vorteilhafte Weiterbildungen des Verfahrens ergeben sich aus dem Betrieb der hierin beschriebenen Injektionsvorrichtung und den dabei offenbar werdenden Merkmalen.

Die Erfindung wird nun anhand von Figuren beschrieben. Die dabei offenbarten Merkmale bilden je einzeln und in Kombination die Erfindung vorteilhaft weiter. Es zeigen:
- Figuren 1A und 1B: Schnittdarstellungen einer Injektionsvorrichtung in einer Ausgangsposition, wobei Figur 1B eine um 90° um die Längsachse gedrehte Ansicht der Figur 1A ist,
- Figuren 2A und 2B: Schnittdarstellungen der Injektionsvorrichtung aus den Figuren 1A und 1B mit einer sich in einer Betätigungsposition befindlichen Betätigungshülse, wobei Figur 2B eine um 90° um die Längsachse gedrehte Ansicht der Figur 2A ist, und
- Figuren 3A und 3B: Schnittdarstellungen der Injektionsvorrichtung aus den Figuren 1A und 1B in einer Einstechposition, wobei Figur 3B eine um 90° um die Längsachse gedrehte Ansicht der Figur 3A ist.

Für die in der Figurenbeschreibung verwendeten Bezugszeichen gilt, dass gleiche Bezugszeichen gleiche Teile bezeichnen.

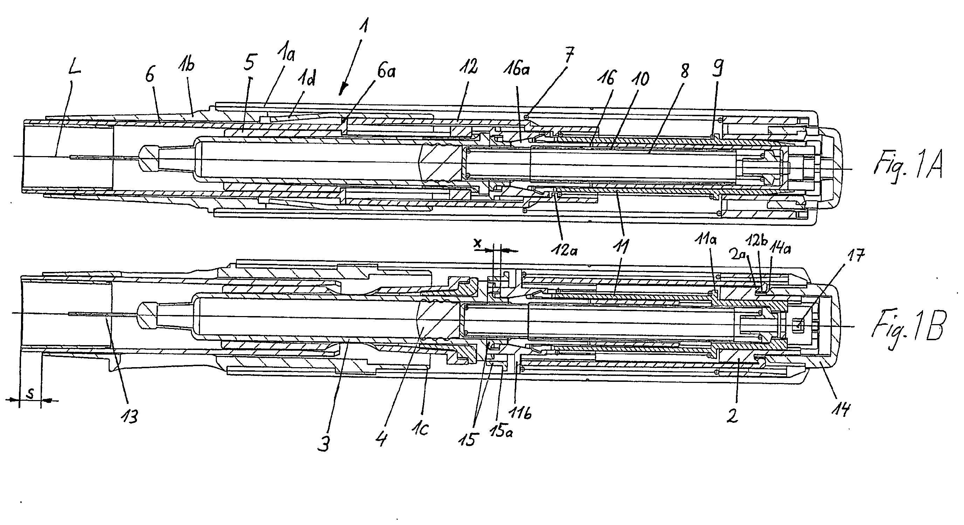

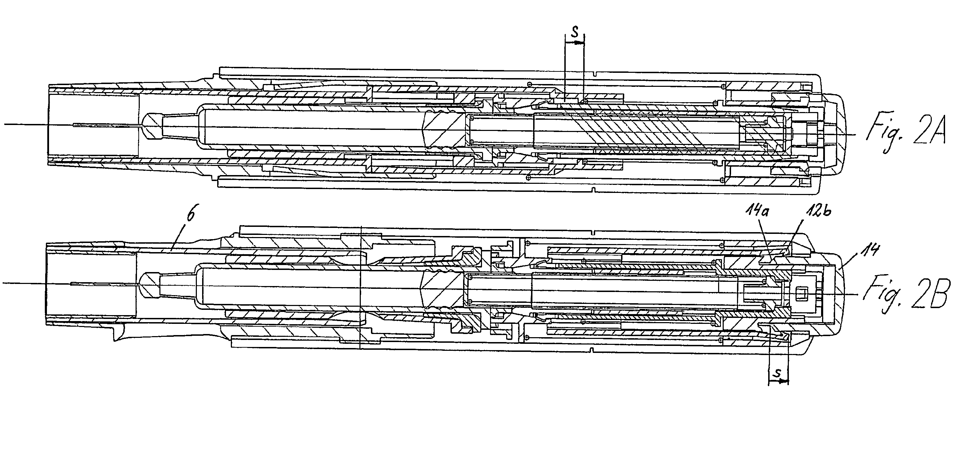

Die Figuren 1A und 1B zeigen eine als Autoinjektor ausgestaltete Injektionsvorrichtung. Die Injektionsvorrichtung umfasst ein Gehäuse 1, welches aus einem distalen Gehäuseteil 1b und einem proximalen Gehäuseteil 1a gebildet wird. Das distale Gehäuseteil 1b ist mit dem proximalen Gehäuseteil 1a axial fest verrastet und vorzugsweise vom Verwender nicht ohne weiteres lösbar. Die zweiteilige Gestaltung der Injektionsvorrichtung hat den Vorteil, dass eine lichtere Montage möglich ist. Von dem Gehäuse 1, insbesondere dem distalen Gehäuseteil 1b wird ein als Nadelschutzhülse 6 ausgestaltetes Betätigungselement axial verschiebbar gelagert, d.h., dass die Nadelschutzhülse 6 entlang der Längsachse L der Injektionsvorrichtung verschiebbar ist. Die Injektionsvorrichtung wird in den Figuren 1A und 1B mit einer sich in ihrer Ausgangsposition befindlichen Nadelschutzhülse 6 gezeigt. In der Ausgangsposition steht die Nadelschutzhülse 6 über das distale Ende des distalen Gehäuseteils 1b über. Wie in diesem Beispiel gezeigt, steht die Nadelschutzhülse 6 um das Maß s über. Eine vom Gehäuse 1 umgebene Injektionsnadel 13 befindet sich mit ihrem distalen Ende proximal des distalen Endes der Nadelschutzhülse 6 und, wie hier gezeigt, auch des distalen Endes des distalen Gehäuseteils 1b. Somit kann auch bei einer um den Weg s in proximale Richtung verschobenen Nadelschutzhülse 6 das distale Ende der Injektionsnadel 13 nicht über das distale Ende der Injektionsvorrichtung hervortreten (Figuren 2A und 2B), d.h. die Spitze der Injektionsnadel ist in der betätigten Position der Nadelschutzhülse 6 vor z.B. versehentlichen Zugriffen geschützt.

Die Nadelschutzhülse 6 ist dafür vorgesehen, an eine Injektionsstelle eines Patienten angesetzt zu werden. Beim Andrücken der angesetzten Injektionsvorrichtung an den Patienten wird die Nadelschutzhülse 6 betätigt, d.h. in proximale Richtung verschoben. Dabei wird ein am proximalen Ende der Nadelschutzhülse 6 anstoßendes Übertragungselement 12 um den gleichen Weg s, um den die Nadelschutzhülse 6 verschoben wird, mitgenommen und damit ebenfalls relativ zum Gehäuse 1 bewegt.

Am Übertragungselement 12 ist ein radial nach innen ragender Kragen 12a gebildet, an dem sich eine Feder 9 mit ihrem distalen Ende abstützt. Mit ihrem proximalen Ende stützt sich die Feder 9 an einem radial nach außen weisenden Kragen 11 a ab, der an einem als Vortriebsglied dienenden Vorschubelement 11 gebildet ist. Somit wird bei der Bewegung der Nadelschutzhülse 6 in seine Betätigungsposition die Feder 9 von der Bewegung des Übertragungselements 12 um den ersten Weg, um einen ersten Betrag vorgespannt. Wird die Nadelschutzhülse 6 beispielsweise um den Weg s in proximale Richtung bewegt, so wird die Feder 9 um einen vom Weg s abhängigen Betrag vorgespannt. Die sich dabei ergebende Federkraft lässt sich beispielsweise über das Hookesche Gesetz ermitteln.

Wenn der Verwender die Injektionsvorrichtung von der Injektionsstelle abnimmt wird durch die Feder 9 die Nadelschutzhülse 6 wieder in ihrer Ausgangsposition, wie sie zum Beispiel in den Figuren 1A und 1B gezeigt wird, zurückgeschoben, da noch keine Einstechbewegung stattgefunden hat.

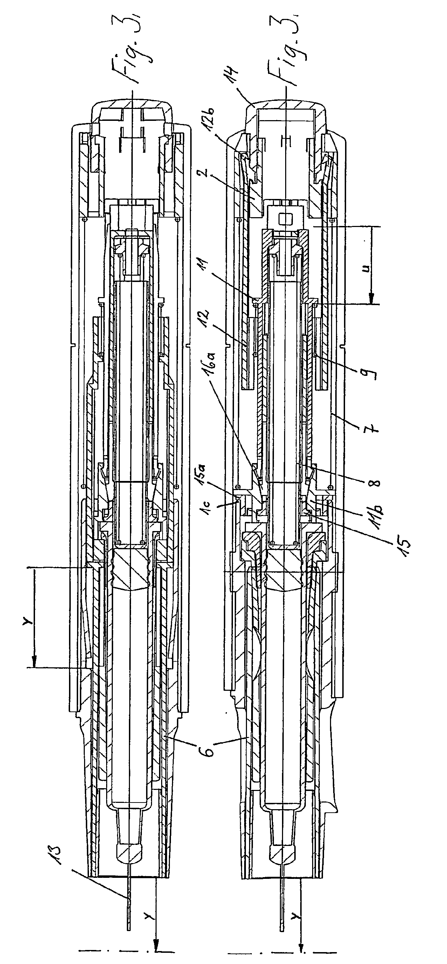

Das Übertragungselement 12 weist an seinem proximalen Ende ein Sperrglied 12b auf, welches in eine Sperrnut eingreift und somit eine Bewegung der Nadelschutzhülse 6 noch weiter in distale Richtung als in den Figuren 1A und 1B dargestellt, verhindert. Die Sperrnut ist distal von einem gehäusefesten Element, insbesondere Käfig 2, und proximal von einer Getriebefläche 14a, die von einem Auslöseelement 14 gebildet wird, begrenzt. Das Auslöseelement 14 dient dazu, die Injektionsvorrichtung für eine Verabreichung des Produkts auszulösen. In der in Figur 1B gezeigten Stellung kann das Auslöseelement 14 nicht gedrückt werden, da der Käfig 2 eine Hintergreifung 2a aufweist, die ein Ausrasten des Sperrglieds 12 mit einer Bewegung radial nach außen verhindert. Die Getriebefläche 14a kann daher trotz Ausübung eines Drucks auf das Auslöseelement 14 das Sperrglied 12b nicht aus der Sperrnut radial nach außen drücken. Wenn die Nadelschutzhülse 6, wie in den Figuren 2A und 2B dargestellt, um den Weg s in proximale Richtung bewegt wird, wird auch das Sperrglied 12b in proximale Richtung bewegt und dabei von der Getriebefläche 14a des Auslöseelements 14 aus der Sperrnut radial nach außen gedrückt. Somit ist das Auslöseelement 14 für eine Auslösebewegung, d.h. für eine Bewegung in distale Richtung, freigegeben. Wenn das Auslöseelement 14 in Auslöserichtung bewegt wird, wird eine Schnappverbindung 17, welche eine Bewegung des Vorschubelements 11 in distale Richtung, d.h. Einstechrichtung, verhindert, freigegeben, so dass letztlich das Vorschubelement 11 in Einstechrichtung bewegbar ist. Nach dem Betätigen des Auslöseelements 14 verrastet es, wie in Figur 3B dargestellt wird, axial fest mit dem Käfig 2, alternativ auch mit dem Gehäuse 1a, so dass die Sperrnut für das Sperrglied 12b soweit geschlossen ist, dass das Sperrelement 12b nicht mehr zwischen die Getriebefläche 14a und der Hintergreifung 2a einrasten kann.

Da nach Betätigen des Auslöseelements 14 das Vorschubelement 11 für eine Bewegung in distale Richtung freigegeben ist, kann eine vorgespannte als Vortriebsfeder dienende Einstechfeder 7 das Vorschubelement 11 in Ausschüttrichtung bewegen. Das Vorschubelement 11 ist mit einem Stützelement 11b axial fest verrastet, so dass sich die beiden Teile wie ein einziges Teil verhalten. Die Zweiteilung ist jedoch aus Montagegründen zweckmäßig. Die Vorschubfeder 7 stützt sich mit ihrem distalen Ende an einem am Stützelement 11b gebildeten, radial nach außen weisenden Kragen und mit ihrem proximalen Ende an dem Käfig 2 ab. Die Vorschubfeder 7 umgibt, wie zum Beispiel in Figur 1B dargestellt, das Übertragungselement 12 und das Vorschubelement 11. Angetrieben durch die Vorschubfeder 7 wird das Vorschubelement 11 mitsamt einer in ihr aufgenommenen Kolbenstange 10 in Ausschüttrichtung bewegt. Hierbei wird die Feder 9 um einen zweiten Betrag, der abhängig vom Weg u ist, zusätzlich zum ersten Betrag, der abhängig vom Weg s ist, gespannt. Hierbei erhöht sich die Federkraft der Feder 9.

Das Vorschubelement 11 ist über das Stützelement 11b so mit einem Produktbehältnis 3 gekoppelt, dass es bei einer Bewegung in Ausschüttrichtung das Produktbehältnis 3 ebenfalls in Ausschüttrichtung mitnehmen kann. Insbesondere stößt das Stützelement 11b mit seinem distalen Ende an dem proximalen Ende des Produktbehältnisses 3 an. Das Produktbehältnis 3 ist in einer Produktbehältnishalterung 5 gehalten. Das Produktbehältnis 3 ist relativ zur Produktbehältnishalterung 5 axial fest gehalten. Insbesondere dient die Produktbehältnishalterung 5 als axiale Führung für das Produktbehältnis 3. Am distalen Ende des Produktbehältnisses 3 ist die Injektionsnadel 13 befestigt. Die Injektionsnadel 13 ist mit dem inneren des Produktbehältnisses 3 fluidisch verbunden. Am proximalen Ende des Produktbehältnisses 3 ist ein Kolben 4 angeordnet, der relativ zum Produktbehältnis 3 bewegbar ist. Bei einer Bewegung des Kolbens 4 in Richtung Injektionsnadel 13 wird das Produkt durch die Injektionsnadel 13 ausgeschüttet.

Bei der Einstechbewegung des Vortriebsgliedes 11 wird das Produktbehältnis 3 und somit die Injektionsnadel 13 in distale Richtung verschoben. Hierbei tritt, wie in den Figuren 3A und 3B dargestellt, die Injektionsnadel 13 distal über das distale Ende der Injektionsvorrichtung hervor. Der Weg, den die Injektionsnadel 13 hervortritt, entspricht bevorzugt der Einstechtiefe in das Gewebe des Patienten.

Vor einer vollständig erfolgten Einstechbewegung ist die von dem Vorschubelement 11 umgebene Kolbenstange 10 axial fest mit dem Vorschubelement 11 verbunden. Das heißt, dass die Kolbenstange 10 die Vorschubbewegung des Vorschubelements 11 mitmacht, wobei keine Relativbewegung zwischen dem Vorschubelement 11 und der Kolbenstange 10 stattfindet. Für die axial feste Anordnung der Kolbenstange 10 relativ zum Vorschubelement 11 ist das Freigabeglied 16a zuständig. Das Freigabeglied 16a ist an einer Funktionshülse 16 gebildet, die zwischen der Kolbenstange 10 und dem Vorschubelement 11 angeordnet ist. Bevorzugt ist das Freigabeglied 16a über einen federnden Arm elastisch mit der Funktionshülse 16 verbunden oder einstückig gebildet. In den in den Figuren 1A,. 1B, 2A und 2B gezeigten Schaltstellungen greift das Freigabeglied 16a mit einem radial nach innen gerichteten Vorsprung in einen an der Außenfläche der Kolbenstange 10 umfänglich gebildeten Kragen oder Absatz ein. Hierdurch wird eine Bewegung der Kolbenstange 10 relativ zum Vorschubelement 11 in distale Richtung verhindert. Das Freigabeglied 16a weist an seiner radial nach außen weisenden Seite eine konusförmige Fläche auf, die mit einer entsprechend entgegenwirkenden, radial nach innen gerichteten konusförmigen Fläche des Stützelements 11b zusammenwirkt. Das Stützelement 11b verhindert mit seiner radial nach innen gerichteten Fläche ein Ausrasten des Freigabeglieds 16a aus dem Eingriff mit der Kolbenstange 10, indem eine Bewegung des Freigabeglieds 16a in radiale Richtung nach außen verhindert wird.

Die Injektionsvorrichtung verfügt ferner über ein Schaltelement 15, welches, wie in Figur 1B dargestellt, um einen Weg x relativ zu dem Stützelement 11b bewegbar ist. Das Schaltelement 15 weist eine Anschlagfläche 15a auf, die mit einem vom Gehäuse 1, insbesondere vom distalen Gehäuseteil 1b gebildeten Anschlag 1c, in einen Axialanschlag bewegbar ist. Am Ende der Einstechbewegung wird das Schaltelement 15 von der Einstechfeder 7 gegen den Anschlag 1c gedrückt. Hierdurch wird das Schaltelement 15 axial fest zum Gehäuse 1 gehalten, während die Einstechfeder 7 das Stützelement 11b weiter in distale Richtung schiebt, wodurch das Stützelement 11b relativ zum Schaltelement 15 um den Weg x, d.h. einen Schaltabstand, verschoben wird. In Figur 3B ist die relative Position des Stützelements 11b und des Schaltelements 15 zueinander gezeigt. Wie ebenfalls in Figur 3B gezeigt wird, besteht zwischen dem Stützelement 11b und dem Freigabeglied 16a nun ein radialer Abstand, so dass das Freigabeglied 16a radial nach außen aus dem Eingriff mit der Kolbenstange 10 ausrasten kann. Die Kolbenstange 10 ist nun für eine Bewegung in Ausschüttrichtung, d.h. distale Richtung, freigegeben.

Die Kolbenstange 10 ist als Hülse gebildet, in der eine Ausschüttfeder 8 zum Antrieb der Kolbenstange 10 relativ zu dem Vorschubelement 11 bzw. für eine Produktausschüttung angeordnet ist. Die Ausschüttfeder 8 stützt sich mit ihrem distalen Ende an einer distal gebildeten Stirnfläche der Kolbenstange 10 und mit ihrem proximalen Ende an dem Vorschubelement 11 oder einem axial fest mit dem Vorschubelement 11 gebildeten Element oder alternativ an der Funktionshülse 16 oder einem axial fest zum Funktionselement 16 gebildeten Element ab. Nach Freigabe der Kolbenstange 10, insbesondere nach der vollständig erfolgten Einstechbewegung, treibt die vorgespannte Ausschüttfeder 8 die Kolbenstange 10 in Ausschüttrichtung an. Die Kolbenstange 10 wirkt mit ihrem distalen Ende auf den Kolben 4, so dass er in distale Richtung mitgenommen wird und eine Produktausschüttung verursacht.

Die hierin beschriebenen Federn sind vorzugsweise Druckfedern und wendelförmig ausgestaltet. Insbesondere sind die Federn aus einem geeigneten metallischen Material, wie zum Beispiel Federstahl, oder einem Kunststoff gebildet. Die Vorspannung bzw. die Auswahl der Einstechfeder 7 ist so gewählt, dass sie in ihrem entspanntesten Zustand eine größere oder zumindest gleich große Federkraft aufweist, wie die Feder 9 in ihrem gespanntesten Zustand, insbesondere nach einer Vorspannung um den ersten Betrag und den zweiten Betrag.

Nach erfolgter Produktausschüttung (in den Figuren nicht dargestellt) nimmt der Verwender der Vorrichtung die Injektionsvorrichtung von der Einstechstelle ab. Hierdurch wird aufgrund der um den ersten Weg s und um den zweiten Weg u vorgespannten Feder 9 die Nadelschutzhülse 6 nun mit einer größeren Kraft in distale Richtung verschoben, als ursprünglich zum Verschieben der Nadelschutzhülse 6 aus der in Figur 1B gezeigten Position um den Weg s in die in Figur 2B gezeigte Position erforderlich war. Da nun die Sperrnut zwischen Käfig 2 und Auslöseelement 14 für ein Einrasten des Sperrglieds 12b verschlossen ist (siehe Figur 3B) kann das Übertragungselement 12 und somit auch die Nadelschutzhülse 6 um einen Weg in distale Richtung verschoben werden, der größer ist als der Weg s, um den die Nadelschutzhülse 6 aus der in Figur 1B gezeigten Position in proximale Richtung verschoben wurde. Vorzugsweise wird die Nadelschutzhülse 6 so weit in distale Richtung verschoben, dass ihr distales Ende distal über das distale Ende der Injektionsnadel 13 ragt. Hierzu ist bevorzugt, dass, wie in Figur 3A gezeigt, ein Abstand zwischen einem an der Nadelschutzhülse 6 radial nach außen gebildeten Verriegelungsnocken 6a und einem vom Gehäuse 1, insbesondere vom distalen Gehäuseteil 1b, gebildeten Axialanschlag ein Abstand y entsprechend gewählt wird. Bei der Bewegung der Nadelschutzhülse 6 in distale Richtung schlägt der Verriegelungsnocken 6a an dem vom Gehäuse 1 gebildeten Axialanschlag an. In dieser Position befindet sich die Nadelschutzhülse 6 in ihrer Endposition, in der es die Injektionsnadel 13 vorzugsweise vollständig abdeckt. Bei der Bewegung des Verriegelungsnockens 6a um den Weg y überfährt der Verriegelungsnocken 6a eine von dem Gehäuse 1, insbesondere dem distalen Gehäuseteil 1b gebildete Verriegelungszunge 1d. Die Verriegelungszunge 1d kann radial nach außen federn und ist vorzugsweise einteilig an dem distalen Gehäuseteil 1b gebildet. Nachdem der Verriegelungsnocken 6a die Verriegelungszunge 1d überfahren hat, federt die Verriegelungszunge 1d radial nach innen, so dass sie sich mit ihrer distalen Anschlagfläche an die proximale Seite des Verriegelungsnockens 6a stemmt. Die Nadelschutzhülse 6 ist nun gegen eine Bewegung in proximale Richtung gesperrt, so dass die Nadel 13 nicht mehr freigegeben werden kann. Mit anderen Worten verrastet die Nadelschutzhülse 6 axial fest mit dem Gehäuse 1. Die Nadelschutzhülse 6 dient dem Nadelschutz und senkt die Verletzungsgefahr für Personen, die mit der Injektionsvorrichtung hantieren. Die gebrauchte Injektionsvorrichtung kann nun sicher entsorgt werden.

### Bezugszeichenliste

- 1: Gehäuse
- 1a: proximales Gehäuseteil
- 1b: distales Gehäuseteil
- 1c: Axialanschlag
- 1d: Verriegelungszunge
- 2: Käfig
- 2a: Hintergreifung
- 3: Produktbehältnis
- 4: Kolben
- 5: Produktbehältnishalter
- 6: Betätigungselement / Nadelschutzhülse
- 7: Einstechfeder
- 8: Ausschüttfeder
- 9: Feder / Rückstellfeder
- 10: Kolbenstange
- 11: Vorschubelement
- 11a: Kragen
- 11b: Stützelement
- 12: Übertragungselement
- 12a: Anschlag / Kragen
- 12b: Sperrglied
- 13: Injektionsnadel
- 14: Auslöseelement
- 14a: Getriebefläche
- 15: Schaltelement
- 15a: Anschlag
- 16: Funktionshülse
- 16a: Freigabeglied
- 17: Schnappverbindung

- s: Vorspannweg 1
- u: Vorspannweg 2
- x: Schaltabstand
- y: Verriegelungsweg
- L: Längsachse

## Patentansprüche

1. injektionsvorrichtung, umfassend
a) ein Gehäuse (1)
b) ein relativ zum Gehäuse (1) verschiebbares Betätigungselement (6), das aus einer Anfangsposition in eine proximale Position verschiebbar ist,
c) eine Feder (9) die mit dem Betätigungselement (6) so gekoppelt ist, dass mit der Feder (9) das Betätigungselement (6) aus einer proximalen Position distal in eine Endposition verschiebbar ist, und
d) ein relativ zum Gehäuse (1) verschiebbares Vortriebsglied,
e) wobei die Feder (9) so mit dem Vortriebsglied gekoppelt ist, dass sie bei einer Bewegung des Vortriebsglieds in Vortriebsrichtung spannbar ist, und
f) ein die Verabreichung des Produkts auslösendes Auslöseelement (14), **gekennzeichnet dadurch, dass**
g) proximal vom Betätigungselement (6) ein Übertragungselement (12) mit einem Sperrglied (12b) angebracht ist und
h) das Sperrglied (12b) in eine Spermut eingreift zum eine Bewegung des Betätigungselements (6) und des Auslöseelements (14) in distale Richtung zu verhindem

2. Injektionsvorrichtung nach einem der vorhergehenden Ansprüche, wobei das Betätigungselement (6) eine Nadelschutzhülse ist, die in ihrer Endposition über das distale Ende einer an der Injektionsvorrichtung gebildeten Injektionsnadel (13) steht.

3. Injektionsvorrichtung nach einem der vorhergehenden Ansprüche, wobei das Betätigungselement (6) aus einer Anfangsposition, in der das Betätigungselement (6) distal über das distale Ende des Gehäuses (1) steht, in die proximale Richtung in die proximale Position, insbesondere in eine Betätigungsposition, bewegbar ist.

4. Injektionsvorrichtung nach dem vorhergehenden Anspruch, wobei die Feder (9) bei der Bewegung des Betätigungselements (6) aus seiner Anfangsposition in die proximale Position um einen ersten Betrag spannbar ist und bei der Bewegung des Vortriebsglieds aus seiner Ausgangsposition in Vortriebsrichtung um einen zweiten Betrag spannbar ist.

5. Injektionsvorrichtung nach einem der vorhergehenden Ansprüche, wobei das Auslöseelement 14 eine Getriebefläche (14a) aufweist.

6. Injektionsvorrichtung nach einem der vorhergehenden Ansprüche, wobei sich die Spermut an einem gehäusefesten Element (2) befindet.

7. Injektionsvorrichtung nach einem der vorhergehenden Ansprüche, wobei über die proximale Verschiebung des Betätigungselements (6) das Sperrglied (12b) von der Getriebefläche (14a) des Auslöseelements (14) aus der Sperrnut radial nach aussen verschiebbar ist.

8. Injektionsvorrichtung nach einem der vorhergehenden Ansprüche, wobei das Betätigungselement (6) in seiner Endposition relativ zum Gehäuse (1) axial fest verrastet ist.

9. Injektionsvorrichtung nach einem der vorhergehenden Ansprüche, ferner umfassend ein Produktbehältnis (3), wobei mit dem Vortriebsglied ein in Produktbehältnis (3) angeordneter Kolben (4) relativ zum Gehäuse (1) verschiebbar ist.

10. Injektionsvorrichtung nach Anspruch 9, wobei das Vortriebsglied ein Vorschubelement (11) ist, mit dem das Produktbehältnis (3) relativ zum Gehäuse (1) verschiebbar ist.

11. Injektionsvorrichtung nach dem vorhergehenden Anspruch, wobei das Produktbehältnis (3) aus einer Anfangsposition distal in eine Endposition verschiebbar ist, wobei in der Endposition eine am Produktbehältnis (3) angeordnete Injektionsnadel (13) distal über das distale Ende der Injektionsvorrichtung hervorsteht.

12. Injektionsvorrichtung nach einem der Ansprüche 9 bis 11, ferner umfassend eine Kolbenstange (10), die relativ zu dem Vorschubelement (11) und mit der der Kolben (4) relativ zum Produktbehältnis (3) verschiebbar ist.

13. Injektionsvorrichtung nach dem vorhergehenden Anspruch, ferner umfassend ein Freigabeglied (16a), welches die Relativbewegung zwischen Kolbenstange (10) und Vorschubelement (11) sperren kann.

14. Injektionsvorrichtung nach einem der vorhergehenden Ansprüche, wobei sich die Feder (9) an dem Vortriebsglied und an dem Betätigungselement (6) oder an einem mit dem Betätigungselement (6) verbundenen Element, insbesondere einem Übertragungselement (12) abstützt.

15. Injektionsvorrichtung nach einem der vorhergehenden Ansprüche, ferner umfassend eine Vortriebsfeder zur Bewegung des Vortriebsglieds in Vortriebsrichtung, wobei mittels der Vortriebsfeder die Feder (9) spannbar ist.

16. Injektionsvorrichtung nach dem vorhergehenden Anspruch, wobei die Vortriebsfeder eine Einstechfeder (7) oder eine Ausschüttfeder (8) oder beides ist.

17. Verfahren zum Betrieb einer Injektionsvorrichtung, wobei ein Vortriebsglied für eine Einstechbewegung oder eine Produktausschüttung relativ zu einem Gehäuse (1) in eine Vortriebsrichtung bewegt wird und dabei eine auf ein Betätigungselement (6) wirkende Feder (9) spannt, wobei das Betätigungselement (6) von der gespannten Feder (9) aus seiner proximalen Position in eine Endposition verschoben wird und wobei ein Auslöseelement die Verabreichung des Produkts auslöst **gekennzeichnet dadurch, dass** ein Übertragungselement (12) mit einem Sperrglied (12b) in eine Sperrnut eingreift und eine Bewegung des Betätigungselements und des Auslöseelements in distale Richtung verhindert.

18. Verfahren nach dem vorhergehenden Anspruche wobei eine injektionsvorrichtung nach einem der Ansprüche 1 bis 16 betrieben wird.

## Claims

1. An injection device comprising
a) a housing (1),
b) an actuating element (6) which is displaceable relative to the housing (1) and which is displaceable from an initial position into a proximal position,
c) a spring (9) so coupled to the actuating element (6) that the actuating element (6) is displaceable from a proximal position distally into an end position by the spring (9), and
d) an advance member displaceable relative to the housing (1),
e) wherein the spring (9) is so coupled to the advance member that it can be stressed upon a movement of the advance member in the advance direction, and
f) a triggering element (14) for triggering the administration of the product,
**characterised in that**
g) a transfer element (12) having a locking member (12b) is mounted proximally from the actuating element (6), and
h) the locking member (12b) engages into a locking groove to prevent a movement of the actuating element (6) and the triggering element (14) in the distal direction.

2. An injection device according to claim 1 wherein the actuating element (6) is a needle projection sleeve which in its end position projects beyond the distal end of an injection needle (13) formed on the injection device.

3. An injection device according to one of the preceding claims wherein the actuating element (6) is movable from an initial position in which the actuating element (6) projects distally beyond the distal end of the housing (1) in the proximal direction into the proximal position, in particular into an actuating position.

4. An injection device according to the preceding claim wherein the spring (9) can be stressed upon the movement of the actuating element (6) from its initial position into the proximal position by a first amount and can be stressed upon the movement of the advance member from its starting position in the advance direction by a second amount.

5. An injection device according to one of the preceding claims wherein the triggering element (14) has a drive surface (14a).

6. An injection device according to one of the preceding claims wherein the locking groove is on an element (2) fixed with respect to the housing.

7. An injection device according to one of the preceding claims wherein the locking member (12b) is displaceable radially outwardly out of the locking groove by the drive surface (14a) of the triggering element (14) by way of the proximal displacement of the actuating element (6).

8. An injection device according to one of the preceding claims wherein the actuating element (6) is axially fixedly latched in its end position relative to the housing (1).

9. An injection device according to one of the preceding claims and further including a product container (3), wherein a piston (4) arranged in the product container (3) is displaceable relative to the housing (3) with the advance member.

10. An injection device according to claim 9 wherein the advance member is a forward thrust element (11) with which the product container (3) is displaceable relative to the housing (1).

11. An injection device according to the preceding claim wherein the product container (3) is displaceable from an initial position distally into an end position, wherein in the end position an injection needle (13) on the product container (3) projects distally beyond the distal end of the injection device.

12. An injection device according to one of claims 9 to 11 and further including a piston rod (10) which is displaceable relative to the forward thrust element (11) and with which the piston (4) is displaceable relative to the product container (3).

13. An injection device according to the preceding claim and further including a release member (16a) which can block the relative movement between the piston rod (10) and the forward thrust element (11).

14. An injection device according to one of the preceding claims wherein the spring (9) is supported against the advance member and the actuating element (6) or against an element connected to the actuating element (6), in particular a transfer element (12).

15. An injection device according to one of the preceding claims and further including an advance spring for moving the advance member in the advance direction, wherein the spring (9) can be stressed by means of the advance spring.

16. An injection device according to the preceding claim wherein the advance spring is an injection spring (7) or an expulsion spring (8) or both.

17. A method of operating an injection device, wherein an advance member for an injection movement or product expulsion is moved relative to a housing (1) in an advance direction and in that case a spring (9) acting on an actuating element (6) is stressed, wherein the actuating element (6) is displaced by the stressed spring (9) from its proximal position into an end position and wherein a triggering element triggers the administration of the product, **characterised in that** a transfer element (12) engages with a locking member (12b) into a locking groove and prevents a movement of the actuating element and the triggering element in the distal direction.

18. A method according to the preceding claim wherein an injection device according to one of claims 1 to 16 is operated.

## Revendications

1. Dispositif d'injection comportant
a) un carter (1)
b) un élément d'actionnement (6) pouvant être coulissé par rapport au carter (1) depuis une position initiale vers une position proximale,
c) un ressort (9) qui est couplé à l'élément d'actionnement (6) de telle manière que le ressort (9) permet de faire coulisser l'élément d'actionnement (6) depuis une position proximale distalement vers une position finale, et
d) un organe de propulsion pouvant être coulissé par rapport au carter (1),
e) le ressort (9) étant couplé à l'organe de propulsion de telle manière que le ressort peut être sollicité lorsque l'organe de propulsion est déplacé dans une direction de propulsion, et
f) un élément de déclenchement (14) déclenchant l'administration du produit, **caractérisé en ce que**
g) un élément de transmission (12) avec un organe de blocage (12b) est placé de manière proximale par rapport à l'élément d'actionnement (6), et **en ce que**
h) l'organe de blocage (12b) vient en prise dans une rainure de blocage afin d'empêcher un déplacement de l'élément d'actionnement (6) et de l'élément de déclenchement (14) dans la direction distale.

2. Dispositif d'injection selon la revendication 1, dans lequel l'élément d'actionnement (6) est une gaine de protection d'aiguille, qui lorsqu'elle se trouve dans sa position finale dépasse de l'extrémité distale d'une aiguille d'injection (13) formée sur le dispositif d'injection.

3. Dispositif d'injection selon l'une quelconque des revendications précédentes, dans lequel l'élément d'actionnement (6) peut être déplacé depuis une position initiale, dans laquelle l'élément d'actionnement (6) dépasse distalement de l'extrémité distale du carter (1), dans la direction proximale dans une position proximale, en particulier dans une position d'actionnement.

4. Dispositif d'injection selon la revendication précédente, dans lequel le ressort (9) peut être sollicité selon un premier niveau de sollicitation lorsque l'élément d'actionnement (6) est déplacé depuis sa position initiale dans la position proximale, et être sollicité selon un deuxième niveau de sollicitation lorsque l'organe de propulsion est déplacé depuis sa position initiale dans la direction de propulsion.

5. Dispositif d'injection selon l'une quelconque des revendications précédentes, dans lequel l'élément de déclenchement (14) présente une surface d'entraînement (14a).

6. Dispositif d'injection selon l'une quelconque des revendications précédentes, dans lequel la rainure de blocage se trouve au niveau d'un élément solidaire du carter (2).

7. Dispositif d'injection selon l'une quelconque des revendications précédentes, dans lequel l'organe de blocage (12b) peut être coulissé radialement vers l'extérieur en dehors de la rainure de blocage par l'intermédiaire de la surface d'entraînement (14a) de l'élément de déclenchement (14) lors du déplacement proximal par coulissement de l'élément d'actionnement (6).

8. Dispositif d'injection selon l'une quelconque des revendications précédentes, dans lequel l'élément d'actionnement (6) dans sa position finale est bloqué de manière fixe dans la direction axiale par rapport au carter (1).

9. Dispositif d'injection selon l'une quelconque des revendications précédentes, comportant en outre un réservoir de produit (3), un piston (4) disposé dans le réservoir de produit (3) pouvant être coulissé par rapport au carter (1) avec l'organe de propulsion.

10. Dispositif d'injection selon la revendication 9, dans lequel l'organe de propulsion est un élément d'avance (11), qui permet de faire coulisser le réservoir de produit (3) par rapport au carter (1).

11. Dispositif d'injection selon la revendication précédente, dans lequel le réservoir de produit (3) peut être coulissé distalement depuis une position initiale vers une position finale, une aiguille d'injection (13) agencée au niveau du réservoir de produit (3) faisant saillie, dans la position finale, distalement au-delà de l'extrémité distale du dispositif d'injection.

12. Dispositif d'injection selon l'une quelconque des revendications 9 à 11, comportant en outre une tige de piston (10) qui peut être coulissée par rapport à l'élément d'avance (11) et qui permet de faire coulisser le piston (4) par rapport au réservoir de produit (3).

13. Dispositif d'injection selon la revendication précédente, comportant en outre un organe de libération (16a) qui peut bloquer le déplacement relatif entre la tige de piston (10) et l'élément d'avance (11).

14. Dispositif d'injection selon l'une quelconque des revendications précédentes, dans lequel le ressort (9) s'appuie au niveau de l'organe de propulsion et au niveau de l'élément d'actionnement (6) ou au niveau d'un élément relié à l'élément d'actionnement (6), en particulier au niveau d'un élément de transmission (12).

15. Dispositif d'injection selon l'une quelconque des revendications précédentes, comportant en outre un ressort de propulsion servant à déplacer l'organe de propulsion dans une direction de propulsion, le ressort (9) pouvant être sollicité au moyen du ressort de propulsion.

16. Dispositif d'injection selon la revendication précédente, dans lequel le ressort de propulsion est un ressort de piqûre (7) ou un ressort de déversement (8).

17. Procédé servant à faire fonctionner un dispositif d'injection, dans lequel un organe de propulsion est déplacé dans une direction de propulsion pour un mouvement de piqûre ou un déversement de produit par rapport à un carter (1) et ce faisant, il sollicite un ressort (9) agissant sur un élément d'actionnement (6), l'élément d'actionnement (6) étant coulissé par le ressort (9) sollicité depuis sa position proximale dans une position finale et dans lequel un élément de déclenchement déclenche l'administration du produit, **caractérisé en ce qu'**un élément de transmission (12) avec un organe de blocage (12b) vient en prise avec une rainure de blocage et empêche un déplacement de l'élément d'actionnement et de l'élément de déclenchement dans la direction distale.

18. Procédé selon la revendication précédente, dans lequel on utilise un dispositif d'injection selon l'une quelconque des revendications 1 à 16.
